(19) Europäisches Patentamt
European Patent Office
Office européen des brevets

(11) Veröffentlichungsnummer: **0 295 536**
**A2**

# (12) EUROPÄISCHE PATENTANMELDUNG

(21) Anmeldenummer: **88109037.7**

(51) Int. Cl.4: **C07C 29/136 , C07C 31/18**

(22) Anmeldetag: **07.06.88**

(30) Priorität: **13.06.87 DE 3719790**

(43) Veröffentlichungstag der Anmeldung: **21.12.88 Patentblatt 88/51**

(84) Benannte Vertragsstaaten: **AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Rutzen, Horst, Dr.**
**Falkenweg 12**
**D-4018 Langenfeld(DE)**
Erfinder: **Stoll, Gerhard, Dr.**
**Eisenbahnstrasse 29**
**D-7808 Waldkirch 3(DE)**

## (54) Herstellung von langkettigen Polyolen aus nachwachsenden Rohstoffen.

(57) Die Erfindung stellt ein Verfahren zur Herstellung von Alkantriolen und Alkanpolyolen zur Verfügung, das dadurch gekennzeichnet ist, daß man einfach und/oder mehrfach epoxidierte Fettsäuren und/oder Fettsäureester sowie deren Gemische mit kurzkettigen Alkancarbonsäuren in an sich bekannter Weise umsetzt und die erhaltenen Esteralkohole an einem Hydrierkontakt bei erhöhter Temperatur und erhöhtem Druck hydriert. Dadurch kann die maximal mögliche Anzahl von Alkoholgruppen pro Molekül gebildet werden.

EP 0 295 536 A2

## Herstellung von langkettigen Polyolen aus nachwachsenden Rohstoffen

Die vorliegende Erfindung betrifft ein Verfahren zur Herstellung von Alkantriolen und Alkanpolyolen aus epoxidierten Fettsäuren und/oder Fettsäureestern.

Zur Herstellung von langkettigen Polyolen geht man üblicherweise von nativen ungesättigten Fettsäuren oder deren Estern aus, überführt diese mit Kaliumpermanganat, Percarbonsäure oder einem anderen geeigneten Reagenz in die Polyhydroxyfettsäure oder deren Ester und reduziert mit Lithiumalanat oder Natrium zum Polyol (B. Palameta, M. Prostenik, Tetrahedron 19 (10) (1963) 1463; F.J. Julietti et al, Soc. 1960(11) 4514).

Verwendet man Percarbonsäuren zur Hydroxylierung der Doppelbindungen, so entstehen zunächst die Epoxide, die in Gegenwart von Carbonsäuren unter Ringöffnung in die entsprechenden Esteralkohole übergehen. Die Esteralkohole werden im Stand der Technik zu den Polyhydroxycarbonsäuren verseift und mit Lithiumalanat redu ziert. Zweckmäßigerweise wird vor der Reduktion mit einem kurzkettigen Alkohol verestert.

Führt man die Reaktion mit Ricinolsäure als Ausgangsmaterial durch, so erhält man als Endprodukt ein Tetrol. (R. Subbarao, K.T. Achaya, J. Sci. Ind. Res. (India) 19B (1960) 482-84).

Ferner wurden Epoxyfettsäuren mit Lithiumalanat direkt zu Diolen reduziert (E.J. Julietti et al, Chem. Ind. 27 (1960) 874). Bei diesem Verfahren geht jedoch eine potentielle Alkoholgruppe verloren.

Aufgabe der vorliegenden Erfindung ist ein Verfahren zur Herstellung von Alkantriolen und Alkanpolyolen aus einfach und/oder mehrfach epoxidierten Fettsäuren und/oder Fettsäureestern, bei dem die maximale Anzahl der möglichen Alkoholgruppen pro Molekül gebildet wird und Verseifungsschritte überflüssig sind.

Es wurde nun gefunden, daß sich langkettige Polyole besonders einfach und wirtschaftlich herstellen lassen, wenn von epoxidierten ungesättigten Fettsäureestern ausgegangen wird, die bereits im technischen Maßstab zur Verfügung stehen. Die Epoxyfettsäureester werden zunächst mit Ameisensäure oder Essigsäure zweckmäßigerweise in Gegenwart von Katalysatoren (Natriumformiat bzw. -acetat) einige Stunden bei erhöhter Temperatur behandelt. Dabei tritt Ringöffnung zum Esteralkohol ein z. B.:

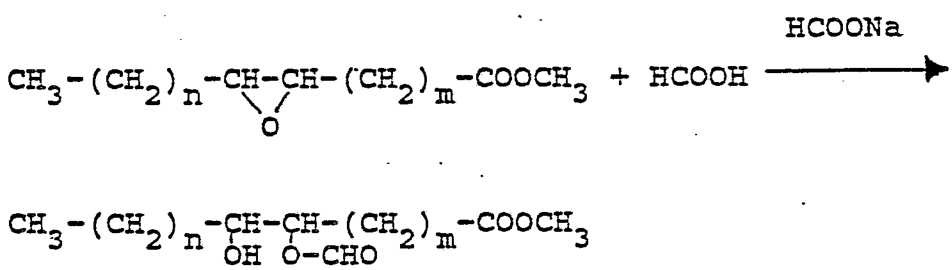


Der Esteralkohol wird nun in einem Schritt katalytisch hydriert, wobei beide Estergruppen gleichzeitig in Alkoholgruppen übergehen:

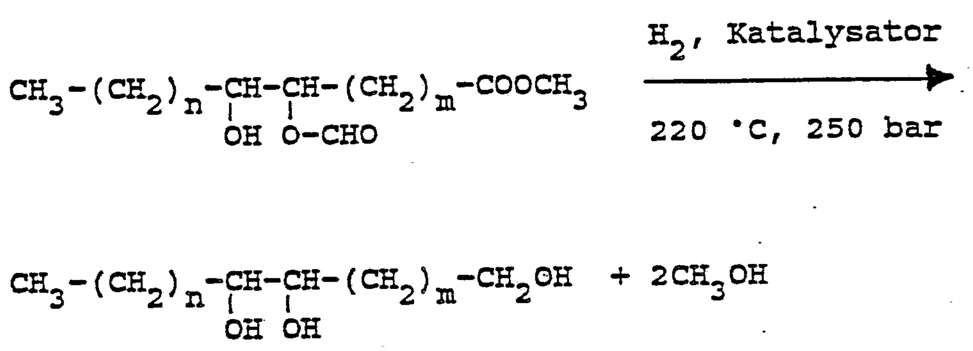


Gegenstand der vorliegenden Erfindung ist ein Verfahren zur Herstellung von Alkantriolen und Alkanpolyolen, das dadurch gekennzeichnet ist, daß man einfach und/oder mehrfach epoxidierte Fettsäuren und/oder Fettsäureester sowie deren jeweilige Gemische mit kurzkettigen Alkancarbonsäuren in an sich bekannter Weise umsetzt und die erhaltenen Esteralkohole an einem Hydrierkontakt bei erhöhter Temperatur und erhöhtem Druck hydriert.

Die für das erfindungsgemäße Verfahren notwendigen epoxidierten Fettsäuren und/oder Fettsäureester

können nach bekannten Verfahren des Standes der Technik aus einfach und/oder mehrfach ungesättigten Fettsäuren und/oder Fettsäureestern hergestellt werden.

Mit Hilfe des vorliegenden Verfahrens ist es möglich, die maximale Anzahl der möglichen Alkoholgruppen pro Molekül zu bilden. Daher sind die mit Hilfe des vorliegenden Verfahrens erhaltenen Polyole ausgezeichnet geeignet als Konsistenzgeber für kosmetische Präparationen sowie auch als Polyolkomponente für die Herstellung von Polyurethanen.

Gemäß einer Ausführungsform der vorliegenden Erfindung werden epoxidierte Fettsäuren und/oder Fettsäureester verwendet, die eine Kettenlänge von 4 bis 24 C-Atomen im Fettsäurerest enthalten. Gleichzeitig weisen die Alkoholkomponenten der Fettsäureester Mono-, Di- und Trialkoholreste mit 1 bis 8 C-Atomen auf. Selbstverständlich können nicht nur einzelne Fettsäuren und/oder Fettsäureester dem erfindungsgemäßen Verfahren unterworfen werden, sondern auch Gemische von epoxidierten Fettsäuren und/oder Gemische von epoxidierten Fettsäureestern sowie die Gemische von epoxidierten Fettsäuren und Fettsäureestern.

Als Ausgangsmaterial gemäß einer Ausführungsform eignen sich beispielsweise die Epoxidierungsprodukte von nativen Ölsäureestern, Sojafettsäureestern, Erucasäureestern, Rübfettsäureestern, Leinfettsäureestern, Safflorfettsäureestern und Baumwollsaatfettsäureestern oder Rindertalg, sowie allgemein die Epoxidierungsprodukte von natürlichen oder synthetischen ungesättigten Fettsäureestern.

Besonders bevorzugt im Sinne der vorliegenden Erfindung sind epoxidierte Fettsäureester, deren Alkoholkomponente sich von Methanol und/oder Glycerin ableiten.

Natürliche ungesättigte Fettsäuren und/oder Fettsäureester, die aus technischem Rindertalg erhalten werden können, weisen üblicherweise eine Doppelbindung auf. Demgemäß besteht eine bevorzugte Ausführungsform der vorliegenden Erfindung darin, daß man einfach epoxidierte Fettsäuren und/oder Fettsäureester sowie deren Gemische einsetzt. Besonders bevorzugt im Sinne der vorliegenden Erfindung ist daher die Verwendung von epoxidiertem technischem Ölsäuremethylester, beispielsweise mit der folgenden Kettenlängenverteilung (Gew.-%):

0 bis 2 % $C_{12}$
2 bis 5 % $C_{14}$
5 bis 7 % $C_{16}$
5 bis 7 % $C_{16}$ einfach ungesättigt
1 bis 3 % $C_{18}$
70 bis 75 % $C_{18}$ einfach ungesättigt
8 bis 12 % $C_{18}$ zweifach ungesättigt
0 bis 1 % $C_{18}$ dreifach ungesättigt und 0 bis 4 % höhere
mit einer Epoxidzahl von 4,6 Gew.-% Epoxidsauerstoff.

Die epoxidierten Fettsäuren und/oder Fettsäureester werden mit Alkancarbonsäuren und/oder deren Alkalimetallsalzen zu den entsprechenden Esteralkoholen umgesetzt. Es ist dabei zweckmäßig, den kurzkettigen Alkohol, mit dem die Epoxidfettsäure verestert ist, und die Carbonsäure, mit dem der Epoxidring geöffnet wurde, so zu wählen, daß nach der Hydrierreaktion ein einheitlicher kurzkettiger Alkohol anfällt. Demgemäß besteht eine Ausführungsform der vorliegenden Erfindung darin, daß die chemische Konstitution der Alkylreste der Alkancarbonsäuren und der Alkoholkomponente der epoxidierten Fettsäureester gleich ist. Beispielsweise können epoxidierte Fettsäuremethylester vorzugsweise mit Ameisensäure umgesetzt werden, wobei die Ameisensäure bei der Hydrierung in Methanol übergeht. Weitere bevorzugte Alkancarbonsäuren im Sinne der Erfindung sind Essigsäure und/oder Propionsäure sowie gegebenenfalls deren Gemische und/oder deren Natrium-und/oder Kaliumsalze. Im Sinne der vorliegenden Erfindung ist jedoch bevorzugt, die Carbonsäure so zu wählen, daß nach der Hydrierreaktion ein einheitlicher kurzkettiger Alkohol anfällt. Demgemäß sind Kombinationen von Methylestern und Ameisensäure, Ethylestern und Essigsäure sowie n-Propylestern und Propionsäure vorteilhaft im Sinne der vorliegenden Erfindung.

Bei Kombination von Methylestern und Essigsäure würde ein Methanol/Ethanol-Gemisch entstehen, das destillativ aufgetrennt werden müßte, sofern keine spezielle Anwendungsmöglichkeit für das spezifische Gemisch bekannt ist.

Der durch die Umsetzung mit einer Alkancarbonsäure entstandene Esteralkohol kann anschließend bei erhöhter Temperatur und erhöhtem Druck an einem geeigneten Hydrierkatalysator, beispielsweise an einem Kupferchromitkatalysator hydriert werden. Gemäß einer bevorzugten Ausführungsform der vorliegenden Erfindung wird ein stückiger kupfer- und/oder zinkhaltiger Katalysator bei Temperaturen zwischen 150 und 300 °C und Drucken zwischen 200 und 300 bar verwendet, wobei ein oxidischer Festbettkatalysator verwendet wird und die Hydrierung mit einem Wasserstoffüberschuß, der das 50 bis 500fache der theoretisch erforderlichen Menge beträgt, gegebenenfalls in Anwesenheit eines Lösungsmittels durchgeführt und das Hydriergas mit einem Umlauf von 1 bis 40 Drucklitern pro Stunde und pro Liter Kontaktvolumen im

Kreislauf geführt wird. Als Lösungsmittel eignen sich beispielsweise aliphatische Alkohole mit 1 bis 4 C-Atomen, insbesondere derartige Alkohole, die auch bei der katalytischen Hydrierung entstehen. Die Herstellung beispielsweise eines bariumhaltigen Kupferchromitkontaktes aus Bariumnitrat, Kupfernitrat und Ammoniumdichromat ist der DE 17 68 313 B2 ebenso zu entnehmen wie die Herstellung eines Kupfer-Zink-Kontaktes aus Zinksulfatheptahydrat und Kupfersulfatpentahydrat. Auf die DE 17 68 313 B2 wird hiermit in bezug auf den erfindungsgemäß verwendeten Katalysator ausdrücklich Bezug genommen. Weiterhin können als Katalysatoren alle diejenigen kupfer-und/oder zinkhaltigen Kontakte verwendet werden, wie sie für die Hydrierung von Fettsäuren zu Fettalkoholen eingesetzt werden. Die Katalysatoren können demnach neben Kupfer und/oder Zink auch weitere Metalle wie beispielsweise Aluminium, Chrom, Vanadin, Wolfram, Molybdän und insbesondere aktivierende Zusätze wie Barium und Cadmium enthalten.

Die Katalysatoren können in kompakter Form, z. B. als Tabletten oder auf geeigneten Trägermaterialien, beispielsweise Bimstein, Kieselgel, Tonerde, natürlichen und synthetischen Zeolithen verwendet werden.

## Beispiele:

### Biespiel 1

324,5 g (1,0 Mol) Epoxystearinsäuremethylester (Epoxidzahl EpOZ 4,93 % daraus ber. Molekulargewicht M 324,5) und 50,6 g (1,1 Mol) Ameisensäure wurden 3 Stunden unter Rühren auf 150 °C gehalten, wobei sich leichter Rückfluß einstellte. Nach der Reaktion war eine klare, gelbe Lösung mit folgenden Kennzahlen entstanden (Säurezahl SZ 28,6; EpOZ 0,0 %. Der Überschuß an Ameisensäure wurde im Ölpumpenvakuum (0,7 mbar) bis zu einer Flüssigkeitstemperatur von 120 °C abdestilliert. Das Endprodukt wog 352,1 g (93,9 %ige Ausbeute)

Kennzahlen:
Hydroxylzahl: OHZ 104
Verseifungszahl: VZ 287
Säurezahl: SZ 4,7
Jodzahl: JZ 5,8

200 g des erhaltenen Esters wurden zusammen mit 40 g eines Kupfer-Chromit-Hydrierkontaktes gemäß Spalte 4 der DE 17 68 313 B2 (A. Herstellung eines bariumhaltigen Kupferchromit-Kontaktes) in einen 1 l-Autoklaven mit Magnethubrührer gegeben und bei 220 °C und 250 bar Wasserstoffdruck innerhalb von 6 Stunden umgesetzt. Der Katalysator wurde im Wärmeschrank bei 100 °C abgesaugt und das Filtrat (127 g) destilliert.

Man erhielt drei Fraktionen:

| | Siedetemp. °C | Druck mbar | Menge g | OHZ | VZ | SZ |
|---|---|---|---|---|---|---|
| Fraktion 1 | 91-155 | 0,01 | 26,1 | 294 | 4,2 | 1,2 |
| Fraktion 2 | 155-195 | 0,01 | 66,3 | 513 | 1,4 | 0,3 |
| Destillationsrückstand | | | 32,8 | 367 | 0,8 | 7,2 |

Fraktion 2 war ein farbloses Kristallisat mit Schmelzgleichgewicht 82 °C.

### Beispiel 2:

341,9 g (1,0 Mol) Epoxystearinsäuremethylester (EpOZ 4,68 % daraus ber. M 341,9) und 66,05 g (1,1 Mol) Essigsäure, ferner 2,44 g Natriumacetat als Katalysator wurden unter Rühren 4 Stunden auf 150 °C gehalten. Nach der Reaktion betrug die SZ 28,7 und die EpOZ 0,083 %. Der Katalysator wurde abfiltriert und die Gesamtmenge (385 g) im Ölpumpenvakuum (0,1 - 0,6 mbar) zwischen 112 und 235 °C überdestilliert. Es verblieben 39,2 g (ca. 10 %) als Rückstand, während 322,2 g als Destillat anfielen.

Die Hydrierung von 320 g des erhaltenen Esters wurde mit 32 g (10 % Kupfer-Chromit-Hydrierkontakt gemäß Beispiel 1 bei 220 °C und 250 bar Wasserstoffdruck innerhalb 8,5 Stunden druchgeführt. Nach Abfiltrieren des Katalysators bei 100 °C wurden 220,1 g Endprodukt mit der OHZ 465 erhalten. 216,1 g wurden zur Destillation im Ölpumpenvakuum eingesetzt.

| | Siedetemp. °C | Druck mbar | Menge g | OHZ | VZ | SZ |
|---|---|---|---|---|---|---|
| Fraktion 1 | 108-180 | 0,05 | 46,6 | 292 | | |
| Fraktion 2 | 180-205 | 0,06 | 158,0 | 495 | 9,0 | 0,2 |
| Destillationsrückstand | | | 7,8 | | | |

Fraktion 2 stellte ein farbloses Kristallisat dar.

**Ansprüche**

1. Verfahren zur Herstellung von Alkantriolen und Alkanpolyolen, dadurch gekennzeichnet, daß man einfach und/oder mehrfach epoxidierte Fettsäuren und/oder Fettsäureester sowie deren jeweilige Gemische mit kurzkettigen Alkancarbonsäuren in an sich bekannter Weise umsetzt und die erhaltenen Esteralkohole an einem Kupferchromitkontakt bei erhöhter Temperatur und erhöhtem Druck hydriert.

2. Verfahren nach Anspruch 1, dadurch gekennzeichnet, daß man epoxidierte Fettsäuren und/oder Fettsäureester verwendet, die eine Kettenlänge von 4 bis 24 C-Atomen im Fettsäurerest enthalten und deren Alkoholkomponente der Fettsäureester aus Mono-, Di- und Trialkoholresten mit 1 bis 8 C-Atomen oder deren Gemische bestehen, verwendet.

3. Verfahren nach Anspruch 2, dadurch gekennzeichnet, daß man epoxidierte Fettsäuren und/oder Fettsäureester verwendet, die sich von Ölsäureestern, Sojafettsäureestern, Erucasäureestern, Rübfettsäureestern, Leinfettsäureestern, Safflorfettsäureestern und Baumwollsaatfettsäureestern oder Rindertalg und deren Gemische ableiten.

4. Verfahren nach Anspruch 2, dadurch gekennzeichnet, daß sich die Alkoholkomponente der Fettsäureester von Methanol und/oder Glycerin ableitet.

5. Verfahren nach den Ansprüchen 1 bis 4, dadurch gekennzeichnet, daß man einfach epoxidierte Fettsäuren und/oder Fettsäureester sowie deren Gemische einsetzt.

6. Verfahren nach den Ansprüchen 1 bis 5, dadurch gekennzeichent, daß man epoxiderte technische Ölsäuremethylester umsetzt.

7. Verfahren nach den Ansprüchen 1 bis 6, dadurch gekennzeichnet, daß die chemische Konstitution der Alkylreste der Alkancarbonsäuren und der Alkoholkomponenten der epoxidierten Fettsäureester gleich ist.

8. Verfahren nach den Ansprüchen 1 bis 7, dadurch gekennzeichnet, daß man als Alkancarbonsäure Ameisensäure, Essigsäure und/oder Propionsäure sowie gegebenenfalls deren Gemische und/oder deren Natrium- und/oder Kaliumsalze verwendet.

9. Verfahren nach den Ansprüchen 1 bis 8, dadurch gekennzeichnet, daß man die Hydrierungsreaktion in Gegenwart stückiger kupfer- und/oder zinkhaltiger Katalysatoren bei Temperaturen zwischen 150 und 350 °C und Drucken zwischen 200 und 300 bar durchführt, wobei oxidische Festbettkatalysatoren eingesetzt werden und die Hydrierung mit einem Wasserstoffüberschuß, der das 50 bis 500fache der theoretisch erforderlichen Menge beträgt, gegebenenfalls in Anwesenheit eines Lösungsmittels durchführt und das Hydriergas mit einem Umlauf von 1 bis 40 Drucklitern pro Stunde und pro Liter Kontaktvolumen im Kreislauf geführt wird.